# EUROPEAN PATENT SPECIFICATION

(11) **EP 3 092 495 B1**
(45) Date of publication and mention of the grant of the patent: **25.11.2020**
(21) Application number: 15733236.2
(22) Date of filing: 06.01.2015
(51) Int. Cl.: G01N 33/68, C07K 16/28, C07K 7/04

(54) **SRM ASSAY FOR PD-L1**
SRM-TEST AUF PD-L1
DOSAGE SRM POUR PD-L1

(30) Priority: 06.01.2014 US 201461924218 P
(43) Date of publication of application: 16.11.2016
(73) Proprietor: Expression Pathology, Inc., Rockville, MD 20850 (US)
(72) Inventor: KRIZMAN, David, B., Gaithersburg, MD 20882 (US); HEMBROUGH, Todd, Gaithersburg, MD 20882 (US); THYPARAMBIL, Sheeno, Frederick, MD 21704 (US); LIAO, Wei-Li, Herndon, VA 20170 (US); AN, Eunkyung, Bethesda, MD 20817 (US)
(74) Representative: AWA Sweden AB
(86) International application number: PCT/US2015/010386
(87) International publication number: WO 2015/103645

(56) References cited:
- WO-A1-2013/173627
- JP-A- 2012 197 258
- US-A1- 2005 048 564
- US-A1- 2012 208 824
- US-B2- 7 833 736
- VELCHETI ET AL.: 'Sarcomatoid Lung Carcinomas Show High Levels of Programmed Death Ligand-1 (PD-L1' J THORAC ONCOL. vol. 8, no. 6, June 2013, ISSN 1556-0864 pages 803 - 805, XP055222355

## Description

### Introduction

Cancer is treated with a collection of therapeutic agents that kill growing and dividing cells and that function in a variety of ways. A common collection of chemotherapeutic agents has been used for decades, either individually or in combinations, and this common collection of agents has become the traditional and routine cancer treatment in clinical oncology practice. Such traditional chemotherapeutic agents act by killing all cells that divide rapidly, one of the main properties of most cancer cells. However, these agents also kill growing normal cells and thus these agents are not considered to be "targeted" approaches to killing cancer cells. In recent years a large group of cancer therapeutic agents have been developed that specifically target only cancer cells where the therapeutic agent specifically attacks a protein that is only expressed by the cancer cells and not normal cells. This approach is considered to be a "targeted" approach to cancer therapy. Most recently, another approach to killing cancer cells in a "targeted" fashion is to specifically modulate the immune system to enhance the ability of the cancer patient's immune system to kill cancer cells.

It is well known that increased expression of PD-L1 by cancer cells may allow those cells to evade the host immune system. Velcheti et al., J Thorac Oncol. 2013 Jun; 8(6); 803-805 discloses that patients with Sarcomatoid carcinomas are positive for PD-L1 and their levels are higher than in conventional non-small-cell lung carcinomas. Renal cell carcinoma where the cancer cells express high levels of PD-L1 is associated with increased tumor aggressiveness and a 4.5-fold increased risk of death. In addition, ovarian cancer patients with higher expression of PD-L1 in their cancer cells have a significantly poorer prognosis than those with lower PD-L1 expression. In these patients, PD-L1 expression correlated inversely with intraepithelial CD8+ T-lymphocyte count, suggesting that PD-L1 expressed on the outside of cancer cells may suppress antitumor activity of CD8+ T cells. This has encouraged development of PD-L1 inhibitors in order to suppress the presence of PD-L1 on the cell surface of cancer cells which, in turn, allows for greater cancer cell killing activity of CD8+ T cells. Based on this recent approach to treating cancer, it is important to understand PD-L1 expression levels in cancer cells to determine whether or not treating a specific cancer with a PD-L1 inhibitor agent will have a significant impact on immune-mediated killing of cancer cells in a given patient.

JP 2012/197258 discloses a tandem mass spectrometry method for quantifying a protein indicative for a disease. WO 2013/173627 discloses a method for measuring the level of the KRT5, KRT7, Napsin A, TTF1, TP63, and/or MUC1 proteins in a biological sample.
Peptides and peptide sequences are provided for use in one or more SRM/MRM assays which are useful for quantitatively determining levels of the PD-L1 protein directly in patient-derived biological samples from cancer patients for improved treatment decisions for cancer therapy.

### Summary

The present invention is defined in the appended claims, directed to a method for measuring the amount of the PD-L1 protein in a human biological sample of formalin-fixed tissue, comprising: detecting and quantifying the amount of a PD-L1 protein fragment peptide in a protein digest prepared from said biological sample using mass spectrometry; and
calculating the amount of PD-L1 protein in said sample;
wherein said PD-L1 fragment peptide is the peptide of SEQ ID NO:4; and
wherein said amount is a relative amount or an absolute amount.

Specific peptides derived from subsequences of the PD-L1 protein are described. PD-L1 is also known as programmed cell death 1 ligand 1, cluster of differentiation 274 (CD274) protein, and B7 homolog 1 (B7-H1). PD-L1 protein is encoded by the CD274 gene, and is referred to herein as PD-L1.

The peptide sequence and fragmentation/transition ions for each peptide derived from proteins are useful in mass spectrometry-based Selected Reaction Monitoring (SRM) assays, which may also be referred to as Multiple Reaction Monitoring (MRM) assays, and which are hereinafter referred to as SRM/MRM assays. The use of peptides for SRM/MRM analysis of the PD-L1 protein and isoforms of this protein is described.

One or more, two or more, three or more, four or more, or five or more SRM/MRM assay(s) can be used to detect the presence and measure *relative* or *absolute* quantitative levels of one or more of the specific peptides derived from cleavage of the PD-L1 protein, and thereby provide a means of measuring the total amount of the PD-L1 protein in a given protein preparation obtained from a biological sample by mass spectrometry. All, or a portion of all, of the available peptides from those proteins can also be analyzed simultaneously in a single SRM/MRM assay or can be analyzed in any combination of individual SRM/MRM assays. Each of the peptides allows measurement of the total amount of the PD-L1 protein in a given protein preparation obtained from a biological sample by mass spectrometry.

The SRM/MRM assay(s) described herein can measure these peptides directly in complex protein lysate samples prepared from cells procured from patient tissue samples, such as formalin fixed cancer patient tissue (*e.g*., resected tumors and biopsies). Methods of preparing protein samples from formalin fixed tissue are described in U.S. Patent No. 7,473,532. The methods described in that patent may conveniently be carried out using Liquid Tissue® reagents and protocol available from Expression Pathology Inc. (Rockville, MD).

Formaldehyde/formalin fixation of tissues surgically removed from cancer patients is the accepted convention in pathology practice. As a result, formaldehyde/formalin fixed paraffin embedded tissue is the most widely available form of tissues from those patients. Formaldehyde/formalin fixation typically employs aqueous solutions of formaldehyde referred to as formalin. "100%" formalin consists of a saturated solution of formaldehyde (about 40% formaldehyde by volume or 37% by mass) in water, with a small amount of stabilizer, usually methanol to limit oxidation and degree of polymerization. The most common way in which tissue is preserved is to soak whole tissue for extended periods of time (8 hours to 48 hours) in aqueous formaldehyde, commonly termed 10% neutral buffered formalin, followed by embedding the fixed whole tissue in paraffin wax for long term storage at room temperature. Thus molecular analytical methods to analyze formalin fixed cancer tissue will be the most accepted and heavily utilized methods for analysis of cancer patient tissue.

Results from the SRM/MRM assay(s) can be used to correlate accurate and precise quantitative levels of any or all of these proteins, in addition to accurate and precise quantitative levels of potential isoforms of these proteins, within specific tissue samples (*e.g*., cancer tissue sample) of a patient or subject from whom the tissue (biological sample) was collected and preserved. This not only provides diagnostic information about the cancer, but also permits a physician or other medical professional to determine appropriate therapy for the patient or subject. Such an assay that provides diagnostically and therapeutically important information about levels of protein expression in a diseased tissue or in another patient/subject sample is termed a *companion diagnostic* assay. For example, such an assay can be designed to diagnose the stage, degree, or histology of a cancer and determine a therapeutic agent that will be most effective in stopping the cancer cells from growing leading to the determination to which therapeutic agent that a patient or subject will most likely respond.

More specifically, detection and/or quantitation of one or more, two or more, three or more, four or more, five or more peptides of the PD-L1 protein, in cancer cells from a patient provides protein information that can indicate which treatment regimen, or regimens, should be followed.

### Detailed Description

The assays described herein quantifying or measure *relative* or *absolute* levels of specific unmodified peptides from the PD-L1 protein and also can measure relative or absolute levels of specific modified peptides from the PD-L1 protein. Examples of modifications include phosphorylated amino acid residues and glycosylated amino acid residues that are present on the peptides.

Relative quantitative levels of proteins and protein isoforms can be determined by the SRM/MRM methodology, for example by comparing SRM/MRM signature peak areas (e.g., signature peak area or integrated fragment ion intensity). Relative levels of individual PD-L1 peptides can be determined in different samples (*e.g*., a control sample and a sample prepared from a patient's or subject's tissue). Alternatively, where each peptide has its own specific SRM/MRM signature peak, it is possible to compare multiple SRM/MRM signature peak areas for one or more of PD-L1 signature peptides. By comparing peak areas it is possible to determine the relative level of the PD-L1 protein and potential protein isoform content in one biological sample or in one or more additional or different biological samples. In this way, the relative amount of a particular peptide, or peptides, from the PD-L1 protein, and therefore the relative amount of the PD-L1 protein, and potential isoforms, can be determined, across multiple (*e.g*., two, three, four, five, or more) biological samples under the same experimental conditions. In addition, relative quantitation can be determined for a given peptide, or peptides, from the PD-L1 protein within a single sample by comparing the signature peak area for that peptide by SRM/MRM methodology to the signature peak area for another and different peptide, or peptides, from a different protein, or proteins, within the same protein preparation from the biological sample.

Using such methodologies the amount of a particular peptide from the PD-L1 protein, and therefore the amount of each of the corresponding proteins and their potential isoforms, can be determined relative one to another within the same sample or in different samples. Since relative quantitation of an individual peptide, or peptides, may be conducted relative to the amount of another peptide, or peptides, within or between samples, it is possible to determine the relative amounts of the peptides present (*e.g*., by determining the peak areas relative one to another), regardless of the absolute weight to volume or weight to weight amounts of the proteins in the biological sample. Thus, the amount of a PD-L1 peptide, or peptides, in the protein preparation from the biological sample may be used to determine the amount of the PD-L1 protein in and among various samples. Relative quantitative data about individual signature peak areas between different samples are generally normalized to the amount of protein analyzed per sample (*e.g*., the total protein concentration of a sample and the volume analyzed are used to normalize samples). Relative quantitation can be performed across many peptides from multiple proteins and the PD-L1 protein simultaneously in a single sample and/or across many samples to gain further insight into relative protein amounts, one peptide/protein with respect to other peptides/proteins.

*Absolute* quantitative levels of the PD-L1 protein are determined by, for example, the SRM/MRM methodology whereby the SRM/MRM signature peak area of an individual peptide from the PD-L1 protein in one biological sample is compared to the SRM/MRM signature peak area of a known amount of one or more internal standards "spiked" in the sample in known amounts (*e.g*., isotope labeled standards). In one embodiment, the internal standard is a synthetic version of the same exact PD-L1 peptide that contains one or more amino acid residues labeled with one or more heavy isotopes. Such isotope-labeled internal standards are synthesized so mass spectrometry analysis generates a predictable and consistent SRM/MRM signature peak that is different and distinct from the native PD-L1 peptide signature peak and which can be used as a comparator peak. Thus, when the internal standard is spiked in known amounts into a protein or peptide preparation from a biological sample in known amounts and analyzed by mass spectrometry, the SRM/MRM signature peak area of the native peptide can be compared to the SRM/MRM signature peak area of the internal standard peptide. The numerical comparison permits a calculation of either the absolute molarity and/or absolute weight of the native peptide present in the original protein preparation from the biological sample, from which the concentration or weight of the corresponding protein may be determined. Absolute quantitative data for fragment peptides are typically displayed according to the amount of protein analyzed per sample. Absolute quantitation can be performed across many peptides, which permits a quantitative determination of multiple proteins (*e.g*., two, three, four, five, etc.) simultaneously in a single sample and/or across multiple samples to gain insight into absolute protein amounts in individual biological samples and/or in entire cohorts of individual samples. In one instance, the quantitation of proteins may be conducted using peptide standards as described by Gygi et al in U.S. Patent 7,501,286.

As used herein the terms quantify, quantifying, measure or measuring mean to determine *relative* or *absolute* levels of an analyte, such as a protein, polypeptide, peptide, a standard (*e.g.,* an internal standard).

Assay methods described herein can be used as an aid for determining the stage of the cancer when employing patient-derived or subject-derived formalin fixed tissue. The SRM/MRM assays described herein may also be used as an aid in determining which therapeutic agent would be most advantageous for use in treating that patient or subject.

To examine the levels of the proteins associated with cancer described herein, analysis can be conducted on cancerous tissue or tissue that is suspected of being cancerous removed from a patient or subject, either through surgical removal of partial or entire tumors, or through biopsy procedures conducted to determine the presence or absence of suspected disease. Samples of the tissues are analyzed to determine whether or not the PD-L1 protein, and which forms of the protein, are present in a patient's or subject's tissue. Moreover, the expression level of the PD-L1 protein can be determined and compared to a "normal" or reference level found in healthy tissue. Normal or reference levels of proteins found in healthy tissue may be derived from, for example, the relevant tissues of one or more individuals that do not have cancer. Alternatively, normal or reference levels may be obtained for individuals with cancer by analysis of relevant tissues (*e.g*., portions of the same organ) not affected by the cancer.

Levels or amounts of proteins or peptides can be defined as the quantity expressed in moles, mass or weight of a protein or peptide determined by the SRM/MRM assay. The level or amount may be normalized to the total level or amount of protein or another component in the lysate analyzed (*e.g*., expressed in micromoles/microgram of protein or micrograms /microgram of protein) or even normalized to the amount of DNA on a per weight basis (*e.g*., micromoles or micrograms/microgram of DNA). In addition, the level or amount of a protein or peptide may be determined on volume basis, expressed, for example, in micromolar or nanograms/microliter. The level or amount of protein or peptide as determined by the SRM/MRM assay can also be normalized to the number of cells analyzed.

Information regarding the PD-L1 protein, and isoforms of this protein, can be used to aid in determining histological stage or grade of a cancer by correlating or comparing the level of the PD-L1 protein, and its isoforms, or fragment peptides with the levels observed in normal tissues. Once the histological stage and/or grade, and/or PD-L1 protein-expression characteristics of the cancer has been determined, that information can be matched to a list of therapeutic agents (chemical and biological) developed to specifically treat cancer tissue that is characterized by, for example, abnormal expression of the protein (*e.g.*, PD-L1) that was assayed. Matching information from the PD-L1 protein assay from a specific individual to a list of therapeutic agents that specifically targets cells/tissue expressing the PD-L1 protein represents a *personalized medicine* approach to treating cancer. The assay methods described herein form the foundation of a *personalized medicine* approach by using analysis of proteins from the patient's or subject's own tissue as a source for diagnostic and treatment decisions.

### Peptide Generation

In principle, any predicted peptide derived from the PD-L1 protein, prepared by any proteolytic process of known specificity may be used as a surrogate reporter to determine the abundance of PD-L1 protein. In practice, however, it is found that only a very few peptides are suitable for use in the assays described herein. Moreover, it is not possible to predict *a priori* which peptide(s), if any, of the enormous number of potential peptides derivable from PD-L1, will be suitable for the assays.

In one embodiment samples are digested with a protease or proteases of known specificity (*e.g.* one or more of trypsin and/or Endoproteinase Lys-C). One or more peptides resulting from the proteolytic treatment can be used as a surrogate reporter to determine the abundance of one or more of PD-L1 protein in a suitable assay such as a mass spectrometry-based SRM/MRM assay. Similarly, any predicted peptide sequence containing an amino acid residue at a site that is known to be modified in the PD-L1 protein may also be used to assay the extent of modification of PD-L1 protein in a sample.

PD-L1 fragment peptides may be generated by a variety of means including by the use of the Liquid Tissue™ protocol provided in US Patent 7,473,532. The Liquid Tissue™ protocol and reagents are capable of producing peptide samples suitable for mass spectroscopic analysis from formalin fixed paraffin embedded tissue by proteolytic digestion of the proteins in the tissue/biological sample. In the Liquid Tissue™ protocol the tissue/biological is maintained at elevated temperatures in a buffer for an extended period of time (*e.g*., from about 80°C to about 100°C for a period of time from about 10 minutes to about 4 hours) to reverse or release protein crosslinking. The buffer employed is a neutral buffer, (*e.g.,* a Tris-based buffer, or a buffer containing a detergent) and advantageously is a buffer that does not interfere with mass spectrometric analysis. Next, the tissue/biological sample is treated with one or more proteases, including but not limited to trypsin, chymotrypsin, pepsin, and Endoproteinase Lys-C for a time sufficient to disrupt the tissue and cellular structure of said biological sample and to liquefy said sample (*e.g*., a period of time from about 30 minutes to about 24 hours at a temperature from about 37°C to about 65°C). The result of the heating and proteolysis is a liquid, soluble, dilutable biomolecule lysate. In one set of embodiment two or more proteases selected from trypsin, chymotrypsin, pepsin, and Endoproteinase Lys-C are employed in the proteolytic treatment of the biological sample.

### Peptide Separation and Assay

Once lysates are prepared, peptides in the samples may be subject to a variety of techniques that facilitate their analysis and measurement (quantification). Where analysis is conducted by mass spectrometry, one or more chromatograph methods may be employed in order to facilitate the analysis.

In one embodiment the peptides are separated by liquid chromatography (LC) prior to analysis by a mass spectrometer instrument. For example, peptides can be separated on an nanoAcquityLC system (Waters, Milford, MA) or EASY-nLC II (Thermo Scientific, San Jose, CA) with a PicoFrit (100µm ID/10µm tip ID, New Objective) column self-packed to a bed length of 12cm with Jupiter Proteo 90Å C12, 4µm resin (Phenomenex, Torrance, CA). Peptides can be eluted over a 12 min chromatography gradient from 1% to 50% acetonitrile, containing 0.1% formic acid and at a flow rate of 800nL/min. Once separated by liquid chromatography, the eluted peptides are directed into a mass spectrometer for analysis. In one embodiment, the mass spectrometer is equipped with a nanospray source.

In another embodiment, the peptides may be separated by an affinity technique such as, for example, immunologically-based purification (*e.g.,* immunoaffinity chromatography), chromatography on ion selective media. If the peptides are modified, separation also can be achieved using appropriate media such as lectins for separation of carbohydrate modified peptides. In still another embodiment, the SISCAPA method, which employs immunological separation of peptides prior to mass spectrometric analysis, is employed. The SISCAPA technique is described, for example, in U.S. Patent No. 7,632,686. In still other embodiments, lectin affinity methods (*e.g.*, affinity purification and/or chromatography may be used to separate peptides from a lysate prior to analysis by mass spectrometry. Methods for separation of groups of peptides, including lectin-based methods, are described, for example, in Geng et al., J. Chromatography B, 752:293-306 (2001). Immunoaffinity chromatography techniques, lectin affinity techniques and other forms of affinity separation and/or chromatography (*e.g*., reverse phase, size based separation, ion exchange) may be used in any suitable combination to facilitate the analysis of peptides by mass spectrometry.

Surprisingly, it was found that many potential peptide sequences from the PD-L1 protein are unsuitable or ineffective for use in mass spectrometry-based SRM/MRM assays for reasons that are not evident. In particular it was found that many tryptic peptides from the PD-L1 protein could not be detected efficiently or at all in a Liquid Tissue™ lysate from formalin fixed, paraffin embedded tissue. As it was not possible to predict the most suitable peptides for MRM/SRM assay, it was necessary to experimentally identify modified and unmodified peptides in actual Liquid Tissue™ lysates to develop a reliable and accurate SRM/MRM assay for the PD-L1 protein. While not wishing to be bound by any theory, it is believed that some peptides might, for example, be difficult to detect by mass spectrometry as they do not ionize well or produce fragments distinct from other proteins. Peptides may also fail to resolve well in separation (*e.g*., liquid chromatography), or adhere to glass or plastic ware. Accordingly, those peptides from the PD-L1 protein that can be detected in a Liquid Tissue™ lysate (*e.g*., the peptides in Tables 1 and 2) prepared from a formalin fixed tissue sample are the peptides for which SRM/MRM assays can be employed in a PD-L1 protein SRM/MRM assay. In one embodiment the protease employed in the simultaneous preparation of fragments of the PD-L1 protein in a single sample will be trypsin.

PD-L1 peptides found in various instances described herein (*e.g*., Tables 1 and/or 2) were derived from the PD-L1 protein by trypsin digestion of all the proteins within a complex Liquid Tissue™ lysate prepared from cells procured from formalin fixed cancer tissue. Unless noted otherwise, in each instance the protease was trypsin. The Liquid Tissue™ lysate was then analyzed by mass spectrometry to determine those peptides derived from the PD-L1 protein that are detected and analyzed by mass spectrometry. Identification of a specific preferred subset of peptides for mass spectrometric analysis is based on: 1) experimental determination of which peptide or peptides from a protein ionize in mass spectrometry analyses of Liquid Tissue™ lysates; and 2) the ability of the peptide to survive the protocol and experimental conditions used in preparing a Liquid Tissue™ lysate. This latter property extends not only to the amino acid sequence of the peptide but also to the ability of a modified amino acid residue within a peptide to survive in modified form during the sample preparation.

Protein lysates from cells procured directly from formalin (formaldehyde) fixed tissue were prepared using the Liquid Tissue™ reagents and protocol that entails collecting cells into a sample tube via tissue microdissection followed by heating the cells in the Liquid Tissue™ buffer for an extended period of time. Once the formalin-induced cross linking has been negatively affected, the tissue/cells are then digested to completion using a protease, such as, in a non-limiting example, the protease trypsin. Each protein lysate is turned into a collection of peptides by digestion of intact polypeptides with the protease. Each Liquid Tissue™ lysate was analyzed (*e.g*., by ion trap mass spectrometry) to perform multiple global proteomic surveys of the peptides where the data was presented as identification of as many peptides as could be identified by mass spectrometry from all cellular proteins present in each protein lysate. An ion trap mass spectrometer or another form of a mass spectrometer that is capable of performing global profiling, for identification of as many peptides as possible from a single complex protein/peptide lysate is typically employed for analysis. Although SRM/MRM assay can be developed and performed on any type of mass spectrometer, including a MALDI, ion trap, or triple quadrupole, the most advantageous instrument platform for SRM/MRM assay is often considered to be a triple quadrupole instrument platform.

Once as many peptides as possible were identified in a single MS analysis of a single lysate under the conditions employed, then that list of peptides was collated and used to determine the proteins that were detected in that lysate. That process was repeated for multiple Liquid Tissue™ lysates, and the very large list of peptides was collated into a single dataset. That type of dataset can be considered to represent the peptides that can be detected in the type of biological sample that was analyzed (after protease digestion), and specifically in a Liquid Tissue™ lysate of the biological sample, and thus includes the peptides for specific proteins, such as for example the PD-L1 protein.

In one instance, the PD-L1 tryptic peptides identified as useful in the determination of absolute or relative amounts of PD-L1 (*e.g.,* NCBI Accession No.: Q9NZQ7, SEQ ID NO:1, SEQ ID NO:2, SEQ ID NO:3, SEQ ID NO:4, SEQ ID NO:5, SEQ ID NO:6, and SEQ ID NO:7), each of which are listed in Table 1. Each of those peptides was detected by mass spectrometry in Liquid Tissue™ lysates prepared from formalin fixed, paraffin embedded tissue. Thus, each of the peptides in Table 1, or any combination of those peptides (*e.g*., one or more, two or more, three or more, four or more, five or more, or six or more of those peptides recited in Table 1) are candidates for use in quantitative SRM/MRM assay for the PD-L1 protein including directly in formalin fixed patient or subject tissue.

**Table 1**

| **SEQ ID** | **Peptide Sequence** |
|---|---|
| SEQ ID NO: 1 | NIIQFVHGEEDLK |
| SEQ ID NO:2 | VQHSSYR |
| SEQ ID NO:3 | DQLSLGNAALQITDVK |
| SEQ ID NO:4 | LQDAGVYR |
| SEQ ID NO: 5 | VNAPYNK |
| SEQ ID NO: 6 | AEVIWTSSDHQVLSGK |
| SEQ ID NO: 7 | TTTTNSKR |

The PD-L1 peptides listed in Table 1 include those detected from multiple Liquid Tissue™ lysates of multiple different formalin fixed tissues of different human organs including prostate, colon, and breast. Each of those peptides is considered useful for quantitative SRM/MRM assay of the PD-L1 protein in formalin fixed tissue. Further data analysis of these experiments indicated no preference is observed for any specific peptides from any specific organ site. Thus, the peptides are suitable for conducting SRM/MRM assays of the PD-L1 protein on a Liquid Tissue™ lysate from any formalin fixed tissue originating from any biological sample or from any organ site in the body.

Compositions comprising peptides that are isotopically labeled, but otherwise identical to one or more of the peptides set forth in this disclosure are provided for herein and their preparation use, particularly for use as mass spectrometry standards, is described below.

In one instance one or more peptides in Table 1, or any combination of those peptides (*e.g*., one or more, two or more, three or more, four or more, five or more, six or more) is assayed by a method that does not rely upon mass spectroscopy, including, but not limited to, immunological methods (*e.g*., Western blotting or ELISA). The assays are conducted using formalin fixed tissue. Regardless of how information directed to the amount of the peptide(s) (absolute or relative) is obtained, the information may be employed in any of the methods described herein, including indicating (diagnosing) the presence of cancer in a patient or subject, determining the stage/grade/status of the cancer, providing a prognosis, or determining the therapeutics or treatment regimen for a patient or subject.

An important consideration when conducting an SRM/MRM assay is the type of instrument that may be employed in the analysis of the peptides. Although SRM/MRM assays can be developed and performed on any type of mass spectrometer, including a MALDI, ion trap, or triple quadrupole, presently the most advantageous instrument platform for SRM/MRM assay is often considered to be a triple quadrupole instrument platform. That type of a mass spectrometer may be considered to be the most suitable instrument for analyzing a single isolated target peptide within a very complex protein lysate that may consist of hundreds of thousands to millions of individual peptides from all the proteins contained within a cell.

In order to most efficiently implement a SRM/MRM assay for each peptide derived from the PD-L1 protein it is desirable to utilize information in addition to the peptide sequence in the analysis. That additional information may be used in directing and instructing the mass spectrometer (*e.g*. a triple quadrupole mass spectrometer) to perform the correct and focused analysis of specific targeted peptide(s) such that the assay may be effectively performed.

The additional information about target peptides in general, and about specific PD-L1 peptides, may include one, two, three, four, or more of the mono isotopic mass of each peptide, its precursor charge state, the precursor m/z value, the m/z transition ions, and the ion type of each transition ion. Additional peptide information that may be used to develop an SRM/MRM assay for the PD-L1 protein is shown in Table 2 for one (1) PD-L1 peptide from the list in Table 1. This additional information described for the peptide as shown in Table 2 may be prepared, obtained, and applied to the analysis of any other peptides from the PD-L1 protein, including those produced by the action of other proteases or combinations of proteases (*e.g*., trypsin and/or Lys C). One, two, three, or four of the transition ions for the peptide of SEQ ID NO:4 may be used for the assay, in any and all combinations.

**Table 2**

| **SEQ ID** | **Peptide Sequence** | **Mono isotopic Mass** | **Precursor Charge State** | **Precursor *m*/*z*** | **Transition *m*/*z*** | **Ion Type** |
|---|---|---|---|---|---|---|
| SEQ ID NO:4 | LQDAGVYR | 920.472 | 2 | 461.243 | 242.149 | b2 |
| | | | 2 | 461.243 | 494.272 | y4 |
| | | | 2 | 461.243 | 565.309 | y5 |
| | | | 2 | 461.243 | 680.336 | y6 |

In some instances, the peptides suitable for assays of PD-L1 protein (e.g., the peptides set forth in Table 1 and shown as SEQ ID Nos. 1-7) may contain additional proteolytic sites internal to the peptide sequences that if cleaved would produce sub-peptides. Such sub-peptides are recognizable by assessing the sequence of the identified peptides for proteolytic cleavage sites of a desired protease. In one embodiment, tryptic peptides may include additional internal trypsin cleavage sites that can lead to sub-peptides upon further cleavage by trypsin. In another embodiment, tryptic peptides may contain internal sites for proteases including, but not limited to, trypsin GluC, AspN, chymotrypsin, and/or Lys C, which can lead to the formation of sub-peptides upon cleavage by any one, two, or more of trypsin, GluC, AspN, chymotrypsin, and/or Lys C. In another embodiment, Lys C peptides may contain internal sites for other proteases, such as GluC, AspN, chymotrypsin, and/or trypsin, which can lead to the formation of sub-peptides upon cleavage by any one, two, or more of GluC, AspN, chymotrypsin, and/or trypsin. Such sub-peptides, and specifically trypsin, GluC, AspN, chymotrypsin, and/or LysC cleavage fragments of any one or more of the peptides set forth in SEQ ID Nos. 1-7 are understood to be set forth in this disclosure.

Instances set forth herein include compositions comprising one or more of the peptides in Tables 1 and 2, and may optionally include peptides that are isotopically labeled but otherwise identical to one or more of the peptides found in Tables 1 and 2. In some instances, the compositions comprise one or more, two or more, three or more, four or more, five or more, six or more, or all seven (7) of the peptides in Tables 1 and 2. Such compositions may optionally include peptides, polypeptides, or proteins whose amino acid sequence comprises peptides that are isotopically labeled but otherwise identical to one or more of the peptides found in Table 1 and Table 2. Where isotopically labeled synthetic or natural peptides, polypeptides, or proteins that comprise one, two, three, four, five, six or more of the peptides in Tables 1 and 2 are employed, protease treatment releases peptides that are isotopically labeled but otherwise identical to the peptides in Tables 1 and 2. Such isotopically labeled biological or biosynthetic peptides may be prepared, for example, in programmed cell lysates or in tissue culture using isotopically labeled amino acids. Each of the isotopically labeled peptides may be labeled with one or more isotopes selected independently from the group consisting of: ¹⁸O, ¹⁷O, ³⁴S, ¹⁵N, ¹³C, ²H or combinations thereof. Compositions comprising peptides from the PD-L1 protein, whether isotope labeled or not, do not need to contain all of the peptides from that protein (*e.g*., a complete set of tryptic peptides). In some instances the compositions do not contain all peptides in combination from the PD-L1 protein, and particularly all of the peptides appearing in Table 1 and Table 2. Compositions comprising peptides may be in the form of dried or lyophilized materials, liquid (*e.g*., aqueous) solutions or suspensions, arrays, or blots.

In one embodiment, the additional information about specific PD-L1 peptides, includes one or more, two or more, or three or more of the mono isotopic mass of each peptide, its precursor charge state, the precursor m/z value, the m/z transition ions, and the ion type of each transition ion for peptides resulting from LysC proteolysis of PD-L1 protein.

In another embodiment, the additional information about specific PD-L1 peptides includes one or more, two or more, or three or more of the monoisotopic mass of each peptide, its precursor charge state, the precursor m/z value, the m/z transition ions, and the ion type of each transition ion for peptides resulting from trypsin proteolysis of PD-L1 protein.

In still another embodiment, the additional information about specific PD-L1 peptides includes one or more, two or more, or three or more of the mono isotopic mass of each peptide, its precursor charge state, the precursor m/z value, the m/z transition ions, and the ion type of each transition ion for peptides resulting from trypsin and/or LysC proteolysis of the PD-L1 protein. In one embodiment, a single tryptic and/or LysC proteolytic peptide from the PD-L1 protein, along with the relevant additional information is employed in a diagnostic determination.

### Certain Examples

Certain examples are described below.
In one example, there is described a method for measuring the level of the PD-L1 protein in a biological sample, comprising detecting and/or quantifying the amount of one or more modified and/or unmodified PD-L1 protein fragment peptides in a protein digest prepared from said biological sample using mass spectrometry; and calculating the level of modified or unmodified PD-L1 protein in said sample; and
wherein said amount is a relative amount or an absolute amount.

The method may further comprise the step of fractionating said protein digest prior to detecting and/or quantifying the amount of one or more modified or unmodified PD-L1protein fragment peptides.

In one example said fractionating step is selected from the group consisting of gel electrophoresis, liquid chromatography, capillary electrophoresis, nano-reversed phase liquid chromatography, high performance liquid chromatography, or reverse phase high performance liquid chromatography.

In one example, said protein digest of said biological sample is prepared by the Liquid Tissue™ protocol.

In one example, said protein digest comprises a protease digest.

In one example, said protein digest comprises a trypsin and/or LysC digest.

In one example, said mass spectrometry comprises tandem mass spectrometry, ion trap mass spectrometry, triple quadrupole mass spectrometry, MALDI-TOF mass spectrometry, MALDI mass spectrometry, and/or time of flight mass spectrometry.

Typically the mode of mass spectrometry used is Selected Reaction Monitoring (SRM), Multiple Reaction Monitoring (MRM), and/or multiple Selected Reaction Monitoring (mSRM), or any combination thereof.

In one example, the PD-L1 protein fragment peptides comprises an amino acid sequence as set forth as SEQ ID NO:1, SEQ ID NO:2, SEQ ID NO:3, SEQ ID NO:4, SEQ ID NO:5, SEQ ID NO:6, and SEQ ID NO:7.

In one example, the biological sample is a blood sample, a urine sample, a serum sample, an ascites sample, a sputum sample, lymphatic fluid, a saliva sample, a cell, or a solid tissue.

In one example, the biological sample is formalin fixed tissue.

In one example, the biological sample is paraffin embedded tissue.

In one example, the biological sample is tissue that is obtained from a tumor.

In one example, the tumor is a primary tumor.

In one example, the tumor is a secondary tumor.

The method may further comprise quantifying modified and/or unmodified PD-L1 protein fragment peptides.

In one example, quantifying the PD-L1protein fragment peptides comprises comparing an amount of one or more PD-L1protein fragment peptides comprising an amino acid sequence of about 8 to about 45 amino acid residues of PD-L1 protein in one biological sample to the amount of the same PD-L1 protein fragment peptides in a different and separate sample or biological sample.

In one example, quantifying the PD-L1 protein fragment peptides comprises comparing an amount of one or more PD-L1 protein fragment peptides comprising an amino acid sequence of about 8 to about 45 amino acid residues of PD-L1 protein, as shown in SEQ ID NO:1, SEQ ID NO:2, SEQ ID NO:3, SEQ ID NO:4, SEQ ID NO:5, SEQ ID NO:6, and SEQ ID NO:7 in one biological sample to the amount of the same PD-L1 protein fragment peptides in a different and separate biological sample.

In one example, quantifying one or more PD-L1 protein fragment peptides comprises determining the amount of the each of the PD-L1 protein fragment peptides in a biological sample by comparison to an added internal standard peptide of known amount, wherein each of the PD-L1 protein fragment peptides in the biological sample is compared to an added internal standard peptide having the same amino acid sequence.

Typically, the internal standard peptide is an isotopically labeled peptide.

Typically, the isotopically labeled internal standard peptide comprises one or more heavy stable isotopes selected from ¹⁸O, ¹⁷O, ³⁴S, ¹⁵N, ¹³C, ²H or combinations thereof.

In one example, detecting and/or quantifying the amount of one or more modified or unmodified PD-L1 protein fragment peptides in the protein digest indicates the presence of modified and/or unmodified PD-L1 protein and an association with cancer in a patient or subject.

The method may further comprise correlating the results of said detecting and/or quantifying the amount of one or more modified and/or unmodified PD-L1 protein fragment peptides, or the amount of said PD-L1 protein to the diagnostic stage/grade/status of the cancer.

In one example, correlating the results of said detecting and/or quantifying the amount of one or more modified or unmodified PD-L1 protein fragment peptides, or the amount of said PD-L1 protein to the diagnostic stage/grade/status of the cancer is combined with detecting and/or quantifying the amount of other proteins or peptides from other proteins in a multiplex format to provide additional information about the diagnostic stage/grade/status of the cancer.

The method may further comprise selecting for a patient or subject from which said biological sample was obtained a treatment based on the presence, absence, or amount of one or more PD-L1 protein fragment peptides or the amount of PD-L1 protein.

The method may further comprise administering to a patient or subject from which said biological sample was obtained a therapeutically effective amount of a therapeutic agent, wherein the therapeutic agent and/or amount of the therapeutic agent administered is based upon amount of one or more modified or unmodified PD-L1 protein fragment peptides or the amount of PD-L1 protein.

In one example, the treatment or the therapeutic agent is directed to cancer cells expressing PD-L1 protein.

In one example, the biological sample is formalin fixed tumor tissue that has been processed for quantifying the amount of one or more modified or unmodified PD-L1 protein fragment peptides employing the Liquid Tissue™ protocol and reagents.

In one example, said one or more modified or unmodified PD-L1 protein fragment peptides is one or more of the peptides in Table 1.

In one example, there is described a composition comprising one or more, two or more, three or more, four or more, five or more, six or more of the peptides in Table 1 and/or antibodies thereto.

### Exemplary SRM/MRM Assay Method

The method described below was used to: 1) identify candidate peptides from the PD-L1 protein that can be used for a mass spectrometry-based SRM/MRM assay for the PD-L1 protein, 2) develop individual SRM/MRM assay, or assays, for target peptides from the PD-L1 protein, and 3) apply quantitative assays to cancer diagnosis and/or choice of optimal therapy.
1. Identification of SRM/MRM candidate fragment peptides for the PD-L1 protein:
   a. Prepare a Liquid Tissue™ protein lysate from a formalin fixed biological sample using a protease or proteases, (that may or may not include trypsin), to digest proteins
   b. Analyze all protein fragments in the Liquid Tissue™ lysate on an ion trap tandem mass spectrometer and identify all fragment peptides from the PD-L1 protein, where individual fragment peptides do not contain any peptide modifications such as phosphorylations or glycosylations
   c. Analyze all protein fragments in the Liquid Tissue™ lysate on an ion trap tandem mass spectrometer and identify all fragment peptides from the PD-L1 protein that carry peptide modifications such as for example phosphorylated or glycosylated residues
   d. All peptides generated by a specific digestion method from the entire, full length PD-L1 protein potentially can be measured, but preferred peptides used for development of the SRM/MRM assay are those that are identified by mass spectrometry directly in a complex Liquid Tissue™ protein lysate prepared from a formalin fixed biological sample
   e. Peptides that are specifically modified (phosphorylated, glycosylated, etc.) in a patient or subject tissue and which ionize, and thus can be detected, in a mass spectrometer when analyzing a Liquid Tissue™ lysate from a formalin fixed biological sample are identified as candidate peptides for assaying peptide modifications of the PD-L1 protein
2. Mass Spectrometry Assay for Fragment Peptides from PD-L1 protein
   a. SRM/MRM assay on a triple quadrupole mass spectrometer for individual fragment peptides identified in a Liquid Tissue™ lysate is applied to peptides from the PD-L1 protein
      i. Determine optimal retention time for a fragment peptide for optimal chromatography conditions including but not limited to gel electrophoresis, liquid chromatography, capillary electrophoresis, nano-reversed phase liquid chromatography, high performance liquid chromatography, or reverse phase high performance liquid chromatography
      ii. Determine the mono isotopic mass of the peptide, the precursor charge state for each peptide, the precursor m/z value for each peptide, the m/z transition ions for each peptide, and the ion type of each transition ion for each fragment peptide in order to develop an SRM/MRM assay for each peptide.
      iii. SRM/MRM assay can then be conducted using the information from (i) and (ii) on a triple quadrupole mass spectrometer where each peptide has a characteristic and unique SRM/MRM signature peak that precisely defines the unique SRM/MRM assay as performed on a triple quadrupole mass spectrometer
   b. Perform SRM/MRM analysis so that the amount of the fragment peptide of the PD-L1 protein that is detected, as a function of the unique SRM/MRM signature peak area from an SRM/MRM mass spectrometry analysis, can indicate both the relative and absolute amount of the PD-L1 protein in a particular protein lysate.
      i. Relative quantitation may be achieved by:
         1. Determining increased or decreased presence of the PD-L1 protein by comparing the SRM/MRM signature peak area from a given PD-L1 peptide detected in a Liquid Tissue™ lysate from one formalin fixed biological sample to the same SRM/MRM signature peak area of the same PD-L1 fragment peptide in at least a second, third, fourth or more Liquid Tissue™ lysate from least a second, third, fourth or more formalin fixed biological sample
         2. Determining increased or decreased presence of the PD-L1 protein by comparing the SRM/MRM signature peak area from a given PD-L1 peptide detected in a Liquid Tissue™ lysate from one formalin fixed biological sample to SRM/MRM signature peak areas developed from fragment peptides from other proteins, in other samples derived from different and separate biological sources, where the SRM/MRM signature peak area comparison between the 2 samples for a peptide fragment are normalized to amount of protein analyzed in each sample.
         3. Determining increased or decreased presence of the PD-L1 protein by comparing the SRM/MRM signature peak area for a given PD-L1 peptide to the SRM/MRM signature peak areas from other fragment peptides derived from different proteins within the same Liquid Tissue™ lysate from the formalin fixed biological sample in order to normalize changing levels of PD-L1 protein to levels of other proteins that do not change their levels of expression under various cellular conditions.
         4. These assays can be applied to both unmodified fragment peptides and for modified fragment peptides of the PD-L1 protein, where the modifications include but are not limited to phosphorylation and/or glycosylation, and where the relative levels of modified peptides are determined in the same manner as determining relative amounts of unmodified peptides.
      ii. Absolute quantitation of a given peptide may be achieved by comparing the SRM/MRM signature peak area for a given fragment peptide from the PD-L1 protein in an individual biological sample to the SRM/MRM signature peak area of an internal fragment peptide standard spiked into the protein lysate from the biological sample
         1. The internal standard is a labeled synthetic version of the fragment peptide from the PD-L1 protein that is being interrogated. This standard is spiked into a sample in known amounts, and the SRM/MRM signature peak area can be determined for both the internal fragment peptide standard and the native fragment peptide in the biological sample separately, followed by comparison of both peak areas
         2. This can be applied to unmodified fragment peptides and modified fragment peptides, where the modifications include but are not limited to phosphorylation and/or glycosylation, and where the absolute levels of modified peptides can be determined in the same manner as determining absolute levels of unmodified peptides.
3. Apply Fragment Peptide Quantitation to Cancer Diagnosis and Treatment
   a. Perform relative and/or absolute quantitation of fragment peptide levels of the PD-L1 protein and demonstrate that the previously-determined association, as well understood in the field of cancer, of PD-L1 protein expression to the stage/grade/status of cancer in patient or subject tumor tissue is confirmed
   b. Perform relative and/or absolute quantitation of fragment peptide levels of the PD-L1 protein and demonstrate correlation with clinical outcomes from different treatment strategies, wherein this correlation has already been demonstrated in the field or can be demonstrated in the future through correlation studies across cohorts of patients or subjects and tissue from those patients or subjects. Once either previously established correlations or correlations derived in the future are confirmed by this assay then the assay method can be used to determine optimal treatment strategy

The internal standard can be a labeled synthetic version of the fragment peptide from the PD-L1 protein that is being interrogated (or a protein or polypeptide comprising the labeled synthetic version of the fragment peptide that is released upon proteolysis). The standard is spiked into a sample in known amounts, and the SRM/MRM signature peak area can be determined for both the internal fragment peptide standard and the native fragment peptide in the biological sample separately, followed by comparison of both peak areas.

This can be applied to unmodified fragment peptides and modified fragment peptides, where the modifications include but are not limited to phosphorylation and/or glycosylation, and where the absolute levels of modified peptides can be determined in the same manner as determining absolute levels of unmodified peptides.

Assessment of PD-L1 protein levels in tissues based on analysis of formalin fixed patient-derived or subject-derived tissue can provide diagnostic, prognostic, and therapeutically-relevant information about each particular patient or subject. Described herein is a method for measuring the levels of the PD-L1 protein in a biological sample, comprising detecting and/or quantifying the amount of one or more modified or unmodified PD-L1 protein fragment peptides in a protein digest prepared from said biological sample using mass spectrometry; and calculating the level of modified or unmodified PD-L1 protein in said sample; and wherein said level is a relative level or an absolute level. In a related embodiment, quantifying one or more PD-L1 protein fragment peptides comprises determining the amount of the each of the PD-L1 protein fragment peptides in a biological sample by comparison to an added internal standard peptide of known amount, wherein each of the PD-L1 protein fragment peptides in the biological sample is compared to an internal standard peptide having the same amino acid sequence. In some embodiments the internal standard is an isotopically labeled internal standard peptide comprises one or more heavy stable isotopes selected from ¹⁸O, ¹⁷O, ³⁴S, ¹⁵N, ¹³C, ²H or combinations thereof.

The method for measuring levels of the PD-L1 protein in a biological sample described herein (or fragment peptides as surrogates thereof) may be used as a diagnostic indicator of cancer in a patient or subject. In one embodiment, the results from measurements of levels of the PD-L1 protein may be employed to determine the diagnostic stage/grade/status of a cancer by correlating (*e.g*., comparing) the levels of PD-L1 protein found in a tissue with the levels of PD-L1 protein found in normal and/or cancerous or precancerous tissues.

The only current method in use for detecting levels of specific proteins in formalin fixed patient tissue is immunohistochemistry (IHC). This method analyzes only one protein at a time on a single tissue section from a patient tumor tissue sample and so, in order to analyze multiple proteins, multiple tissue sections must be interrogated which is time and labor-intensive. IHC uses an antibody to detect the presence of the target protein and, because of the potential for non-specific binding of the antibody to proteins, there is great inherent potential for signal background in any IHC experiment. In addition, IHC is only semi-quantitative at best. Due to these problems IHC fails to provide for objective quantitative analysis of multiple proteins simultaneously. The current embodiment is able to provide for objective quantitation of the PD-L1 protein simultaneously with 100% assay specificity utilizing a single section of a patient tissue sample saving significant time and money while providing for much more valuable data about expression of the PD-L1 protein.

This multiplex SRM/MRM assay can also include simultaneous analysis of other additional proteins beyond the PD-L1 protein, including drug target proteins such as EGFR, IGF-1R, and cMet. This is valuable because analysis of additional proteins also indicates which additional drugs might be useful for treating a particular cancer. Examples of additional drugs based on analysis of additional example drug target proteins include Erbitux, which targets the EGFR receptor, Figitumumab, which targets IGF-1R, and Foretinib, which targets c-Met and vascular endothelial growth factor receptor 2 (VEGFR-2). An example of a drug that targets PD-L1 is an engineered monoclonal anti-PDLl antibody referred to as MPDL3280A or RG7446. This antibody may be used alone or in combination, for example, with Avastin® (bevacizumab) for solid tumors, and with Zelboraf® (vemurafenib) for metastatic melanoma.

Because both nucleic acids and protein can be analyzed from the same Liquid Tissue™ biomolecular preparation it is possible to generate additional information about disease diagnosis and drug treatment decisions from the nucleic acids in same sample upon which proteins were analyzed. For example, if the PD-L1 protein is expressed by certain cells at increased levels, when assayed by SRM the data can provide information about the state of the cells and their potential for uncontrolled growth, potential drug resistance and the development of cancers can be obtained. At the same time, information about the status of the PD-L1 genes and/or the nucleic acids and proteins they encode (*e.g*., mRNA molecules and their expression levels or splice variations) can be obtained from nucleic acids present in the same Liquid Tissue™ biomolecular preparation can be assessed simultaneously to the SRM analysis of the PD-L1 protein. Any gene and/or nucleic acid not from the PD-L1 and which is present in the same biomolecular preparation can be assessed simultaneously to the SRM analysis of the PD-L1 protein. In one embodiment, information about the PD-L1 protein and/or one, two, three, four or more additional proteins may be assessed by examining the nucleic acids encoding those proteins. Those nucleic acids can be examined, for example, by one or more, two or more, or three or more of: sequencing methods, polymerase chain reaction methods, restriction fragment polymorphism analysis, identification of deletions, insertions, and/or determinations of the presence of mutations, including but not limited to, single base pair polymorphisms, transitions, transversions, or combinations thereof.

The above description and exemplary methods and compositions are illustrative of the present disclosure. Because of variations which will be apparent to those skilled in the art, however, the present disclosure is not intended to be limited to the particular embodiments described above.

## Claims

1. A method for measuring the amount of the PD-L1 protein in a human biological sample of formalin-fixed tissue, comprising:
detecting and quantifying the amount of a PD-L1 protein fragment peptide in a protein digest prepared from said biological sample using mass spectrometry; and
calculating the amount of PD-L1 protein in said sample;
wherein said PD-L1 fragment peptide is the peptide of SEQ ID NO:4; and
wherein said amount is a relative amount or an absolute amount.

2. The method of claim 1, further comprising the step of fractionating said protein digest prior to detecting and quantifying the amount of said PD-L1 protein fragment peptide.

3. The method of claim 1, wherein said protein digest comprises a protease digest.

4. The method of claim 1, wherein said mass spectrometry comprises tandem mass spectrometry, ion trap mass spectrometry, triple quadrupole mass spectrometry, MALDI-TOF mass spectrometry, MALDI mass spectrometry, and/or time of flight mass spectrometry.

5. The method of any one of claims 1 to 4, wherein quantifying said PD-L1 protein fragment peptide comprises comparing an amount of said PD-L1 protein fragment peptide in one biological sample to the amount of the same PD-L1 protein fragment peptide in a different and separate biological sample.

6. The method of any one of claims 1 to 4, wherein quantifying said PD-L1 protein fragment peptide comprises determining the amount of said PD-L1 protein fragment peptide in a biological sample by comparison to an added internal standard peptide of known amount, wherein said PD-L1 protein fragment peptide in the biological sample is compared to an internal standard peptide having the same amino acid sequence.

7. The method of claim 6, wherein the internal standard peptide is an isotopically labeled peptide.

8. The method of any of claims 1 to 4, wherein detecting and quantifying the amount of said PD-L1 protein fragment peptide in the protein digest indicates the presence of modified or unmodified PD-L1 protein and an association with cancer in a patient or subject from whom the tissue sample was obtained.

9. The method of claim 8, further comprising correlating the results of said detecting and quantifying the amount of said PD-L1 protein fragment peptide, or the amount of said PD-L1 protein to the diagnostic stage/grade/status of the cancer.

10. The method of claim 9, wherein correlating the results of said detecting and quantifying the amount of said PD-L1 protein fragment peptide, or the amount of said PD-L1 protein to the diagnostic stage/grade/status of the cancer is combined with detecting and/or quantifying the amount of other proteins or peptides from other proteins in a multiplex format to provide additional information about the diagnostic stage/grade/status of the cancer.

11. The method of any one of claims 8 to 10, wherein the cancer is lung cancer.

12. The method of claim 9, further comprising selecting for the patient or subject from whom said biological sample was obtained a therapeutically effective amount of a therapeutic agent, wherein the therapeutic agent and/or amount of the therapeutic agent is based upon the amount of said PD-L1 protein fragment peptide or the amount of PD-L1 protein.

13. The method of claim 12, wherein the therapeutic agent is directed to cancer cells expressing PD-L1 protein.

## Patentansprüche

1. Verfahren zum Messen der Menge des PD-L1-Proteins in einer humanen biologischen Probe von Formalinfixiertem Gewebe, umfassend:
Nachweisen und Quantifizieren der Menge eines PD-L1-Proteinfragmentpeptids in einer Proteinspaltung, hergestellt aus der biologischen Probe unter Verwendung von Massenspektrometrie; und
Berechnen der Menge an PD-L1-Protein in der Probe;
wobei das PD-L1-Fragmentpeptid das Peptid von SEQ ID NO: 4 ist; und
wobei die Menge eine relative Menge oder eine absolute Menge ist.

2. Verfahren nach Anspruch 1, zudem umfassend den Schritt des Fraktionierens der Proteinspaltung vor dem Nachweisen und dem Quantifizieren der Menge des PD-L1-Proteinfragmentpeptids.

3. Verfahren nach Anspruch 1, wobei die Proteinspaltung eine Proteasespaltung umfasst.

4. Verfahren nach Anspruch 1, wobei die Massenspektrometrie Tandem-Massenspektrometrie, Ionenfallen-Massenspektrometrie, Dreifach-Quadrupol-Massenspektrometrie, MALDI-TOF-Massenspektrometrie, MALDI-Massenspektrometrie und/oder Time-of-Flight-Massenspektrometrie umfasst.

5. Verfahren nach einem der Ansprüche 1 bis 4, wobei das Quantifizieren des PD-L1-Proteinfragmentpeptids das Vergleichen einer Menge des PD-L1-Proteinfragmentpeptids in einer biologischen Probe mit der Menge des gleichen PD-L1-Proteinfragmentpeptids in einer anderen und getrennten biologischen Probe umfasst.

6. Verfahren nach einem der Ansprüche 1 bis 4, wobei das Quantifizieren des PD-L1-Proteinfragmentpeptids das Bestimmen der Menge des PD-L1-Proteinfragmentpeptids in einer biologischen Probe durch Vergleichen mit einem zugesetzten internen Standardpeptid bekannter Menge umfasst, wobei das PD-L1-Proteinfragmentpeptid in der biologischen Probe mit einem internen Standardpeptid mit der gleichen Aminosäuresequenz verglichen wird.

7. Verfahren nach Anspruch 6, wobei das interne Standardpeptid ein isotopenmarkiertes Peptid ist.

8. Verfahren nach einem der Ansprüche 1 bis 4, wobei das Nachweisen und Quantifizieren der Menge des PD-L1-Proteinfragmentpeptids in der Proteinspaltung das Vorhandensein von modifiziertem oder nicht modifiziertem PD-L1-Protein und eine Assoziation mit Krebs, bei einem Individuum oder Probanden, aus dem die Gewebeprobe erhalten wurde, anzeigt.

9. Verfahren nach Anspruch 8, zudem umfassend das Korrelieren der Ergebnisse des Nachweises und der Quantifizierung der Menge des PD-L1-Proteinfragmentpeptids oder der Menge des PD-L1-Proteins mit dem diagnostischen Stadium/Grad/Status der Krebserkrankung.

10. Verfahren nach Anspruch 9, wobei das Korrelieren der Ergebnisse des Nachweises und der Quantifizierung der Menge des PD-L1-Proteinfragmentpeptids oder der Menge des PD-L1-Proteins mit dem diagnostischen Stadium/Grad/Status der Krebserkrankung mit dem Nachweis und/oder der Quantifizierung der Menge anderer Proteine oder Peptide aus anderen Proteinen in einem Multiplex-Format kombiniert wird, um zusätzliche Informationen über das diagnostische Stadium/den Grad/den Status der Krebserkrankung bereitzustellen.

11. Verfahren nach einem der Ansprüche 8 bis 10, wobei es sich bei der Krebserkrankung um Lungenkrebs handelt.

12. Verfahren nach Anspruch 9, zudem umfassend das Auswählen einer therapeutisch wirksamen Menge eines Therapeutikums für den Patient oder das Individuum, von dem die biologische Probe erhalten wurde, wobei das Therapeutikum und/oder die Menge des Therapeutikums auf der Menge des PD-L1-Proteinfragmentpeptids oder der Menge an PD-L1-Protein beruht.

13. Verfahren nach Anspruch 12, wobei das Therapeutikum auf Krebszellen gerichtet ist, die PD-L1-Protein exprimieren.

## Revendications

1. Procédé destiné à mesurer la quantité de la protéine PD-L1 dans un échantillon biologique humain de tissu fixé à la formaline, comprenant les étapes consistant à :
détecter et quantifier la quantité d'un peptide d'un fragment protéique de PD-L1 dans un produit de digestion de protéines préparé à partir dudit échantillon biologique en utilisant une spectrométrie de masse ; et
calculer la quantité de protéine PD-L1 dans ledit échantillon ;
dans lequel ledit peptide d'un fragment de PD-L1 est le peptide de SEQ ID n° : 4 ; et
dans lequel ladite quantité est une quantité relative ou une quantité absolue.

2. Procédé de la revendication 1, comprenant en outre l'étape de fractionnement dudit produit de digestion de protéines avant de détecter et de quantifier la quantité dudit peptide d'un fragment protéique de PD-L1.

3. Procédé de la revendication 1, dans lequel ledit produit de digestion de protéines comprend un produit de digestion d'une protéase.

4. Procédé de la revendication 1, dans lequel ladite spectrométrie de masse comprend une spectrométrie de masse en tandem, une spectrométrie de masse à piège ionique, une spectrométrie de masse triple quadripolaire, une spectrométrie de masse MALDI-TOF, une spectrométrie de masse MALDI et/ou une spectrométrie de masse à temps de vol.

5. Procédé de l'une quelconque des revendications 1 à 4, dans lequel la quantification dudit peptide d'un fragment protéique de PD-L1 comprend une comparaison d'une quantité dudit peptide d'un fragment protéique de PD-L1 dans un échantillon biologique avec la quantité du même peptide d'un fragment protéique de PD-L1 dans un échantillon biologique différent et séparé.

6. Procédé de l'une quelconque des revendications 1 à 4, dans lequel la quantification dudit peptide d'un fragment protéique de PD-L1 comprend une détermination de la quantité dudit peptide d'un fragment protéique de PD-L1 dans un échantillon biologique par comparaison avec un peptide standard interne ajouté de quantité connue, dans lequel ledit peptide d'un fragment protéique de PD-L1 dans l'échantillon biologique est comparé à un peptide standard interne ayant la même séquence d'acides aminés.

7. Procédé de la revendication 6, dans lequel le peptide standard interne est un peptide marqué par isotopes.

8. Procédé de l'une quelconque des revendications 1 à 4, dans lequel la détection et la quantification de la quantité dudit peptide d'un fragment protéique de PD-L1 dans le produit de digestion de protéines indique la présence d'une protéine PD-L1 modifiée ou non modifiée et une association à un cancer chez un patient ou un sujet à partir duquel l'échantillon de tissu a été obtenu.

9. Procédé de la revendication 8, comprenant en outre une corrélation des résultats desdites détection et quantification de la quantité dudit peptide d'un fragment protéique de PD-L1, ou de la quantité de ladite protéine PD-L1, au stade/grade/état de diagnostic du cancer.

10. Procédé de la revendication 9, dans lequel la corrélation des résultats desdites détection et quantification de la quantité dudit peptide d'un fragment protéique de PD-L1, ou de la quantité de ladite protéine PD-L1, au stade/grade/état de diagnostic du cancer est combinée avec une détection et/ou une quantification de la quantité d'autres protéines, ou de peptides provenant d'autres protéines, dans un format multiplexé pour fournir des informations supplémentaires à propos du stade/grade/état de diagnostic du cancer.

11. Procédé de l'une quelconque des revendications 8 à 10, dans lequel le cancer est un cancer du poumon.

12. Procédé de la revendication 9, comprenant en outre une sélection, pour le patient ou le sujet à partir duquel ledit échantillon biologique a été obtenu, d'une quantité efficace d'un point de vue thérapeutique d'un agent thérapeutique, dans lequel l'agent thérapeutique et/ou la quantité de l'agent thérapeutique est basé(e) sur la quantité dudit peptide d'un fragment protéique de PD-L1 ou la quantité de protéine PD-L1.

13. Procédé de la revendication 12, dans lequel l'agent thérapeutique est dirigé contre des cellules cancéreuses exprimant une protéine PD-L1.
